(19) Europäisches Patentamt / European Patent Office / Office européen des brevets

(11) EP 3 613 345 A1

(12) EUROPEAN PATENT APPLICATION
published in accordance with Art. 153(4) EPC

(43) Date of publication:
26.02.2020 Bulletin 2020/09

(51) Int Cl.:
A61B 5/1455 (2006.01)    G01N 21/359 (2014.01)

(21) Application number: 18788542.1

(22) Date of filing: 17.04.2018

(86) International application number:
PCT/JP2018/015866

(87) International publication number:
WO 2018/194056 (25.10.2018 Gazette 2018/43)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 18.04.2017 JP 2017082123

(71) Applicant: Kowa Company Ltd.
Naka-ku
Nagoya-shi
Aichi 460-8625 (JP)

(72) Inventor: ASANO, Takaharu
Higashimurayama-shi
Tokyo 189-0022 (JP)

(74) Representative: Ribeiro, James Michael
Withers & Rogers LLP
4 More London Riverside
London SE1 2AU (GB)

(54) INFORMATION PROCESSING METHOD FOR CALCULATING ABSORPTION SPECTRUM OF BLOOD, INFORMATION PROCESSING DEVICE, AND PROGRAM

(57) Provided is an information processing method configured to further accurately calculate an absorption spectrum of blood based on light applied to a living body such as a human finger. The information processing method includes the steps of: irradiating a living body with near-infrared light having a plurality of wavelengths; measuring, based on the near-infrared light received from the living body, a pulsating signal corresponding to each of the plurality of wavelengths; performing a principal component analysis on multi-wavelength time-series data including the measured pulsating signal corresponding to each of the wavelengths so as to remove noise from the pulsating signal; and calculating an absorption spectrum based on a variation width of the pulsating signal that is removed noise.

FIG. 8

**Description**

[Field]

The present invention relates to an information processing method, an information processing device, and a program, each being configured to calculate an absorption spectrum of blood.

[Background]

Blood contains a blood cell, water, and various components such as a protein (e.g., albumin and hemoglobin), neutral fat, cholesterol and glucose, each of which reflects a human health condition. Thus, in order to evaluate a human health condition, it is important to measure a component in blood, and an analysis of the blood collected by using an injection needle is typically performed. However, owing to the factors such as pain caused by an injection needle puncturing, complex handling of procedures (e.g., collecting blood, measuring components in blood, and disposing of the injection needle), and a risk of infection, frequent blood analysis is not practical, and therefore a method to noninvasively measure components in blood without performing blood collection is requested. Accordingly, there has been studied a method to noninvasively obtain an absorption spectrum of blood and analyze the spectrum shape, so as to presume concentration of components in blood.

As a method to noninvasively measure and calculate an absorption spectrum of blood, PTL 1 discloses a method, in which a living body is irradiated with light, and an intensity of the light received from the living body is obtained. On an assumption that the intensity of the light periodically varies in accordance with pulsation of blood, a difference method is applied to the intensities of the light at different time points (e.g., the maximum value and the minimum value), so that a variation width in intensity of the light is obtained. Based on the variation width in intensity of the light, an absorbance of blood is calculated. These steps are applied to the light at each wavelength. As a result, an absorbance of blood at each wavelength, in other words, an absorption spectrum of blood at each wavelength is calculated. However, in this method, random noise is caused by an operation of each of various sensors or others on the device or a fluctuation of the light applied to the living body. The random noise is included in the measurement result of intensity of the light, thereby resulting in an inaccurate variation width in intensity of the light. In this configuration, the absorption spectrum of blood may not accurately be calculated.

PTL 2 proposes a device configured to measure cerebral blood flow by using a principal component analysis which is a method of multivariate analysis. However, the device is configured to accurately measure local cerebral blood flow by performing the principal component analysis on a signal of light at a specified wavelength, the light received from a plurality of parts in a living body. Thus, when the living body is irradiated with light having a plurality of wavelengths and the device is applied to measure an absorption spectrum of blood based on the light, the noise described above is not removed from the measured absorption spectrum.

[Citation List]

[Patent Literature]

[PTL 1] JP-B-3345481
[PTL 2] JP-B-4474145

[Summary]

[Technical Problem]

In view of the respects described above, an object of the present disclosure technique is to provide an information processing method, an information processing device, and a program, each configured to further accurately calculate an absorption spectrum of blood based on light applied to a living body such as a human finger.

[Solution to Problem]

As a result of constant study, the inventor of this application has achieved the present invention by identifying a method that includes the steps of: irradiating a living body with light having a plurality of wavelengths; obtaining, based on the light received from the living body, a temporal variation in intensity or absorbance of the light at each of the wavelengths, in other words, a pulsating signal at each of the wavelengths; preparing multi-wavelength time-series data including the pulsating signal at each of the wavelengths; performing a principal component analysis on the prepared multi-wavelength time-series data to remove noise from the pulsating signal at each of the wavelengths; and calculating a variation width of the pulsating signal at each of the wavelengths that is removed noise.

The present disclosure provides an information processing method configured to calculate an absorption spectrum of blood. The information processing method includes the steps of: irradiating a living body with near-infrared light having a plurality of wavelengths; measuring, based on the near-infrared light received from the living body, a pulsating signal corresponding to each of the plurality of wavelengths; and performing a principal component analysis on multi-wavelength time-series data including the measured pulsating signal corresponding to each of the wavelengths so as to remove noise from the pulsating signal corresponding to each of the wavelengths. In this configuration, the pulsating signal measured based on the light received from the living body is reconstructed as a pulsating signal that maintains a variation in the light attributed only to the pulsation. As a result, an accurate variation width of the pulsating signal at each of the wavelengths is obtained, and thus, an absorbance of blood at each of the wavelengths, i.e., an absorption spectrum of blood is calculated more accurately than in the existing techniques.

In the information processing method, the principal component analysis may be performed to obtain a first principal component, and based on the obtained first principal component, the pulsating signal corresponding to each of the wavelengths may be calculated. In this configuration, since data indicating the variation in the light attributed only to the pulsation is highly likely to appear as the first principal component in a result of the principal component analysis, noise is removed while the data is maintained. The plurality of wavelengths are within a wavelength range of preferably 400 to 2500 nm, and more preferably 900 to 1300 nm.

The present disclosure additionally provides an information processing device configured to calculate an absorption spectrum of blood. The information processing device includes: a measuring unit that measures a pulsating signal corresponding to each of a plurality of wavelengths based on a near-infrared light having the plurality of wavelengths, the near-infrared light received from a living body by irradiating the living body with the near-infrared light; and a calculating unit that performs a principal component analysis on multi-wavelength time-series data including the measured pulsating signal corresponding to each of the wavelengths so as to remove noise from the pulsating signal corresponding to each of the wavelengths. The calculating unit may perform the principal component analysis to obtain a first principal component, and based on the obtained first principal component, calculate the pulsating signal corresponding to each of the wavelengths. The plurality of wavelengths are within a wavelength range of preferably 400 to 2500 nm, and more preferably 900 to 1300 nm.

The present disclosure further provides a program configured to calculate an absorption spectrum of blood. The program causes a computer to perform: measuring a pulsating signal corresponding to each of a plurality of wavelengths based on a near-infrared light having the plurality of wavelengths, the near-infrared light received from a living body by irradiating the living body with the near-infrared light; and performing a principal component analysis on multi-wavelength time-series data including the measured pulsating signal corresponding to each of the wavelengths so as to remove noise from the pulsating signal corresponding to each of the wavelengths. The program may cause the computer to perform the principal component analysis to obtain a first principal component, and based on the obtained first principal component, calculate the pulsating signal corresponding to each of the wavelengths. The plurality of wavelengths are within a wavelength range of preferably 400 to 2500 nm, and more preferably 900 to 1300 nm.

[Advantageous Effects of Invention]

The present disclosure technique provides an information processing method, an information processing device, and a program, each configured to further accurately calculate an absorption spectrum of blood based on light applied to a living body such as a human finger.

[Brief Description of Drawings]

[Fig. 1] Fig. 1 illustrates a configuration example of an in-blood component calculation device according to an embodiment.
[Fig. 2] Fig. 2 illustrates a schematic view of the in-blood component calculation device according to the embodiment.
[Fig. 3] Fig. 3 illustrates a partial schematic view of the in-blood component calculation device according to the embodiment.
[Fig. 4] Fig. 4 is an example flowchart that illustrates processing operations performed by the in-blood component calculation device according to the embodiment.
[Fig. 5] Fig. 5 illustrates a configuration example of a simulated blood vessel test system according to the embodiment.

[Fig. 6] Fig. 6 illustrates an example of multi-wavelength time-series data used in the embodiment.

[Fig. 7] Fig. 7 is an example graph that illustrates a time-averaged transmitted light spectrum obtained over a measurement period in a first example.

[Fig. 8] Fig. 8 is an example graph that illustrates a temporal variation in amount of the transmitted light (a pulsating signal) at wavelength of 1048 nm, with respect to the transmitted light spectrum measured in the first example.

[Fig. 9] Fig. 9 is an example graph that illustrates a temporal variation in amount of the transmitted light (a pulsating signal) at wavelength of 1138 nm, with respect to the transmitted light spectrum measured in the first example.

[Fig. 10] Fig. 10 is an example graph that illustrates a temporal variation in amount of the transmitted light (a pulsating signal) at wavelength of 1195 nm, with respect to the transmitted light spectrum measured in the first example.

[Fig. 11] Fig. 11 is an example graph that illustrates a standard deviation of the temporal variation that is calculated as a variation width in amount of the transmitted light at each of the wavelengths in the transmitted light spectrum in the first example.

[Fig. 12] Fig. 12 is an example graph that illustrates an absorption spectrum measured in the first example.

[Fig. 13] Fig. 13 is an example graph that illustrates a time-averaged transmitted light spectrum obtained over a measurement period in a second example.

[Fig. 14] Fig. 14 is an example graph that illustrates a temporal variation in amount of the transmitted light (a pulsating signal) at wavelength of 1048 nm, with respect to the transmitted light spectrum measured in the second example.

[Fig. 15] Fig. 15 is an example graph that illustrates a temporal variation in amount of the transmitted light (a pulsating signal) at wavelength of 1138 nm, with respect to the transmitted light spectrum measured in the second example.

[Fig. 16] Fig. 16 is an example graph that illustrates a temporal variation in amount of the transmitted light (a pulsating signal) at wavelength of 1195 nm, with respect to the transmitted light spectrum measured in the second example.

[Fig. 17] Fig. 17 is an example graph that illustrates a standard deviation of the temporal variation that is calculated as a variation width in amount of the transmitted light at each of the wavelengths in the transmitted light spectrum in the second example.

[Fig. 18] Fig. 18 is an example graph that illustrates an absorption spectrum measured by irradiating a living body with light in the second example.

[Fig. 19] Fig. 19 is an example graph that illustrates an absorption spectrum measured by irradiating a paper filter with light in the second example.

[Description of Embodiments]

Embodiments of the present invention will be described below with reference to the drawings. In this embodiment, an object of which blood is subjected to calculation of an absorption spectrum, may be a part where blood pulsates, such as preferably a finger, a toe, a palm of hand, a sole of foot, an earlobe, lips, or the like, particularly preferably a finger, and more particularly preferably an index finger.

In this embodiment, a variation width of a pulsating signal at each wavelength of light received from a living body is appropriately converted to an absorbance for calculating the absorption spectrum of blood. For example, when measuring the absorbance of the received light, the variation width of the pulsating signal represents the absorbance of blood. When measuring an intensity of the received light, it is possible to convert the variation width of the pulsating signal ($P_{sd}$) to a variation width of the absorbance (A) by applying a time average of the pulsating signal ($P_{ave}$) and the variation width of the pulsating signal ($P_{sd}$) to a following mathematical formula (1).

[Mathematical formula 1]

$$A = -\log_{10}\left(\frac{p_{ave} - p_{sd}}{p_{ave} + p_{sd}}\right) \cdots (1)$$

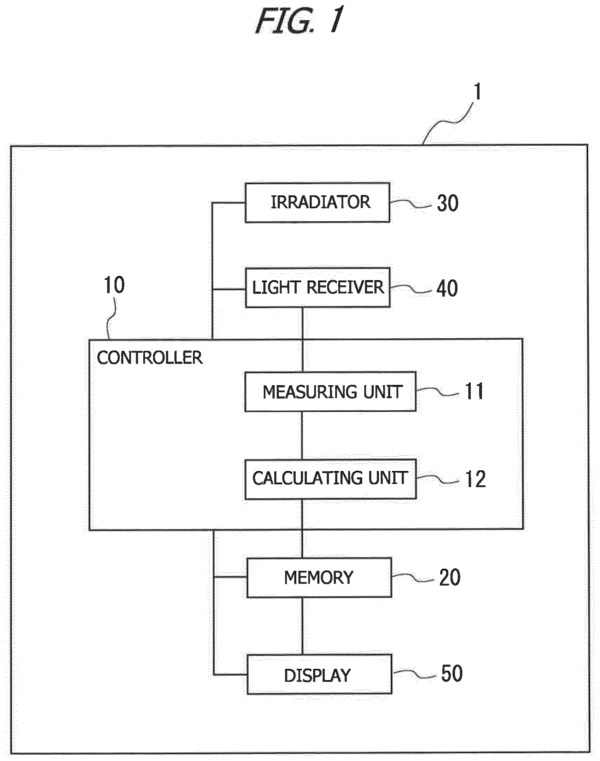

As an example of an information processing device according to this embodiment, Fig. 1 illustrates a schematic configuration example of an in-blood component calculation device 1. The in-blood component calculation device 1, for example, irradiates a part of a subject's body (e.g., a finger), of which blood is subjected to calculation of an absorption spectrum, with near-infrared light. Then, based on a transmitted light spectrum through the part of the subject's body, the absorption spectrum of blood is calculated. As illustrated in Fig. 1, the in-blood component calculation device 1 includes a controller 10, a memory 20, an irradiator 30, a light receiver 40, and a display 50.

The controller 10 controls an operation of each part in the in-blood component calculation device 1. The memory 20 stores programs to cause various processing operations to be performed in the in-blood component calculation device 1, as will be described below. The memory 20 also stores data obtained when the various processing operations are performed in the in-blood component calculation device 1. The controller 10 executes the programs stored in the memory 20 by developing the programs on a Random Access Memory (RAM; not illustrated) in the in-blood component calculation device 1, so that the various processing operations are performed in the in-blood component calculation device 1. The irradiator 30 irradiates a part of the subject's body (e.g., a finger), of which blood is subjected to calculation of the absorption spectrum, with the near-infrared light. Transmitted light that is transmitted through the subject's body is received by the light receiver 40.

The controller 10 includes a measuring unit 11 and a calculating unit 12 as part of functions of the controller 10. The measuring unit measures a pulsating signal based on the transmitted light received by the light receiver 40. The calculating unit 12 removes noise from the measured pulsating signal to calculate the absorption spectrum of the subject's blood. The absorption spectrum of the blood calculated by the calculating unit 12 is displayed on the display 50.

In the embodiment, a multichannel Fourier transform spectrometer for example is used as the light receiver 40 for measuring the near-infrared absorption spectrum. In this device, incoming light is separated by a Savart plate, an interference pattern (interferogram) resulting from interference generated between the separated lights with a Fourier lens is obtained by a line sensor, and the resulting interferogram is Fourier transformed to obtain an optical spectrum. The measurable wavelengths with a multichannel Fourier transform spectrometer include the entire near-infrared range (900 to 2500 nm). The multi-channel Fourier-transform spectrometer is used to measure a transmitted light spectrum of a sample. The transmitted light spectrum of the sample is then compared with a transmitted light spectrum of a blank sample that is previously obtained. As a result, an absorption spectrum of the sample relative to the blank sample is obtained.

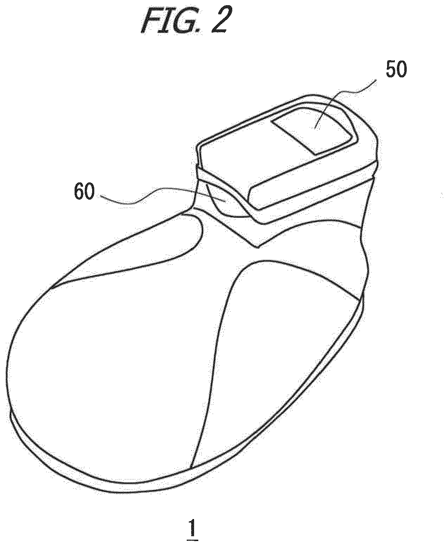

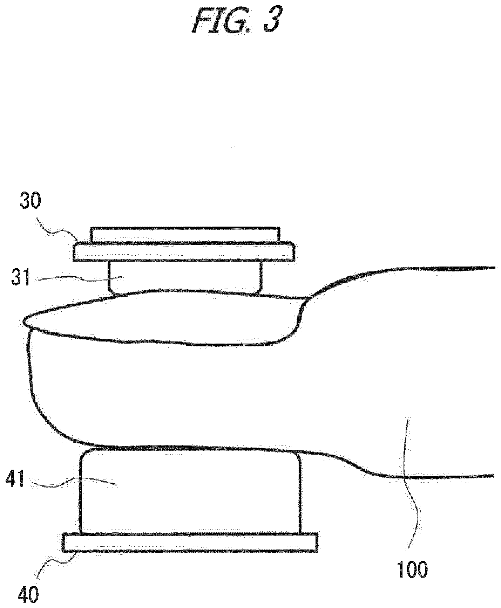

Fig. 2 illustrates a schematic example of the in-blood component calculation device 1 according to this embodiment. The in-blood component calculation device 1 has an opening 60 into which the subject inserts a finger 100. The irradiator 30 and the light receiver 40 are provided at the back of the opening 60. Fig. 3 schematically illustrates a state at which the subject inserts the finger 100 into the opening 60 of the in-blood component calculation device 1 of Fig. 2. The irradiator 30 and the light receiver 40 are disposed to sandwich the finger 100 that the subject inserts from the opening 60.

The irradiator 30 includes a halogen lamp 31. As an example, a wavelength of the light emitted by the halogen lamp 31 is white light in a wavelength range of 900 to 1700 nm. Note that, the irradiator 30 has a light source of which the type (for example, including a Light Emitting Diode (LED)), the number, and the wavelength range are not limited to those described above. The light receiver 40 includes a light detector 41. This configuration causes the near-infrared light, with which the irradiator 30 irradiates the finger 100, to be transmitted through the finger 100 and received by the light receiver 40. In the in-blood component calculation device 1, the absorption spectrum of the subject's blood is calculated based on the near-infrared light received by the light receiver 40, in accordance with the processing operations as will be described below. The calculated absorption spectrum is displayed on the display 50.

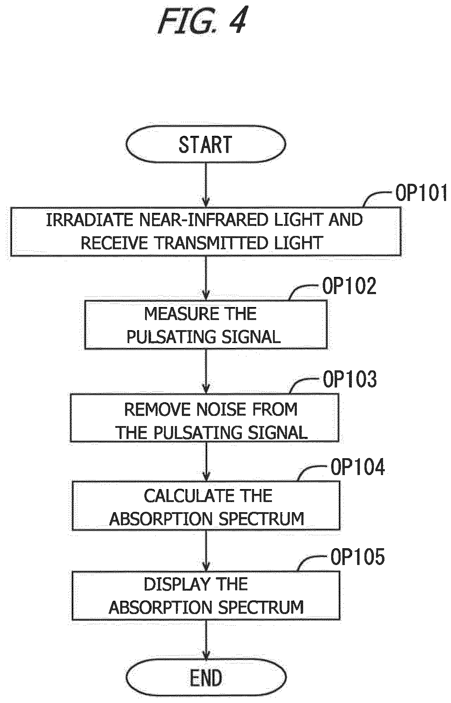

Fig. 4 is an example of a flowchart that illustrates processing operations performed by the controller 10. The controller 10 starts the processing operations of the flowchart illustrated in Fig. 4 in accordance with, for example, an operation by a user of the in-blood component calculation device 1.

In OP 101, the controller 10 controls the irradiator 30 to irradiate a part of the subject's body with the near-infrared light. The near-infrared light irradiated from the irradiator 30 transmits through the finger of the subject and enters the light receiver 40 as the transmitted light. Then, the process moves to OP 102. In the OP 102, the controller 10 measures the pulsating signal, which has been described above, by using the transmitted light received by the light receiver 40.

In OP 103, the controller 10 calculate a pulsating signal removed noise from the pulsating signal measured in the OP 102. The removal of noise will be described in detail later in an example. In OP 104, the controller 10 calculates an absorption spectrum based on a variation width of the pulsating signal that is removed noise as calculated in the OP 103. In OP 105, the controller 10 displays the absorption spectrum calculated in the OP 104 on the display 50 and terminates the processing operations of the flowchart.

First example

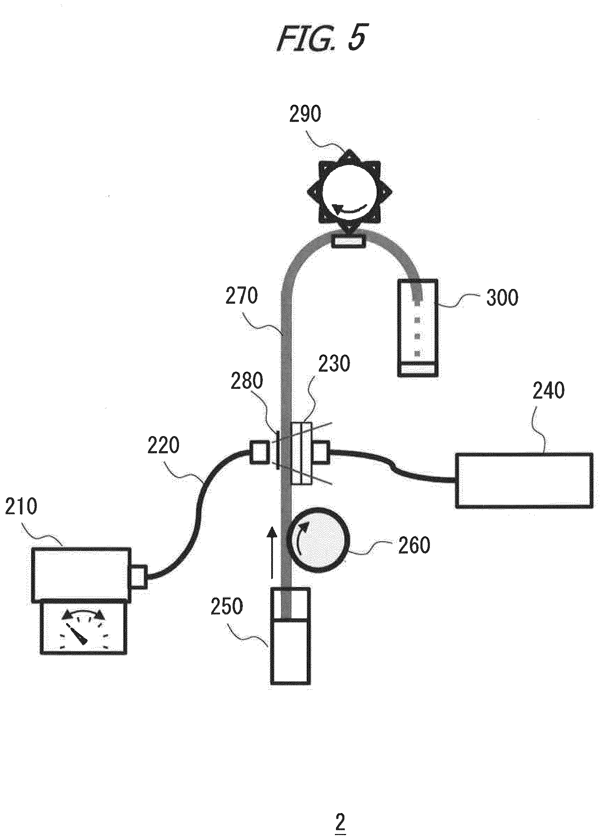

Next, a first example according to this embodiment will be described. In the first example, in order to perform a test to see if the in-blood component calculation device 1 described above noninvasively and accurately calculates an absorption spectrum, a simulated blood vessel test system 2 illustrated in Fig. 5 is used. The simulated blood vessel test system 2 includes a light source 210, a bundle fiber 220, a light receiver 230, a spectrometer 240, a solution tank 250, a diaphragm pump 260, a tube 270, a filter paper 280, a peristaltic pump 290, and a liquid receiving tank 300. The solution tank 250 stores water of which the absorption spectrum is known. The solution tank 250 is connected to one end of the tube 270 such as a Tygon tube. The tube 270 has the other end connected to the liquid receiving tank 300. In a middle of the tube 270, the diaphragm pump 260 is provided closer to the solution tank 250, and the peristaltic pump 290 is provided closer to the liquid receiving tank 300. The diaphragm pump 260 causes the water stored in the solution tank 250 to travel through the tube 270 at a constant flow rate such as approximately 200 ml per minute. The peristaltic pump 290 periodically crushes the tube 270 to generate a pulsating current in the tube 270.

Additionally, the tube 270 has the light receiver 230 disposed at a position between the diaphragm pump 260 and the peristaltic pump 290. The tube 270 has a portion, on which the light receiver 230 is disposed. The portion of the tube 270 is covered with a filter paper 280 as light scattering medium to light applied to the tube 270. In this embodiment, the tube 270 is used as a simulated blood vessel, and a combination of the tube 270 and the filter paper 280 is used as an imitated human finger. The light source 210 is connected to one end of the bundle fiber 220. The light source 210 emits light that passes through the bundle fiber 220 to the other end of the bundle fiber 220, from which the light is applied to the portion of the tube 270 covered with the filter paper 280. The light applied to the portion of the tube 270 covered with the filter paper 280 is received by the light receiver 230 to be sent to the spectrometer 240.

The spectrometer 240 measures a pulsating signal of the water as solution sent through the tube 270. The system subsequently uses a computer (not illustrated) to remove noise from the measured pulsating signal and calculates an absorption spectrum based on a variation width of the pulsating signal that is removed noise. Then, the calculated absorption spectrum is compared with the absorption spectrum of water that is previously known. As a result, the test is performed to see if the system noninvasively and accurately calculates the absorption spectrum of the solution sent through the tube.

In the first example, the diaphragm pump 260 is driven to send the water stored in the solution tank 250 to the tube 270 at the constant flow rate (approximately 200 ml per minute), and the peristaltic pump 290 is driven to generate the pulsating flow. Then, the light source (halogen lamp) emits white light at a wavelength range of 900 to 1700 nm. The white light passes through the bundle fiber 220 and is subsequently applied to the tube 270 covered with the filter paper 280. The light transmitted through the tube 270 covered with the filter paper 280 is received by the light receiver 230. The spectrometer 240 measures the light received by the light receiver 230 at each of 62 different wavelengths within the wavelength range of 900 to 1700 nm for a period of 10 seconds, i.e., 200 times at an interval of 50 milliseconds, so as to obtain an optical spectrum at each of the 62 different wavelengths.

In this embodiment, the absorption spectrum of the water to be sent through the tube is separately measured by placing the water in a container and using air as a blank sample. The simulated blood vessel test system 2 obtains the pulsating signal at each of the wavelengths that constitutes multi-wavelength time-series data. A principal component analysis is performed on the multi-wavelength time-series data, using a Pirouette Multivariate Data Analysis Software version 4.5 (Infometrix, Inc.) as an example. At the principal component analysis, the data centralization based on an average value is performed as a preprocessing operation, and the principal component calculation is made to obtain a first principal component only. In this embodiment, removing noise from the pulsating signal by the principal component analysis is referred to as "reconstructing" the pulsating signal. Further, in this embodiment, with respect to the reconstructed pulsating signal, the variation width of the pulsating signal at each of the wavelengths is calculated as a standard deviation. Then, the variation width of the pulsating signal at each of the wavelengths is converted to a variation width of an absorbance by using the mathematical formula (1) and a time-averaged amount of the transmitted light at each of the wavelengths. As a result, the absorption spectrum of the solution sent through the tube is calculated.

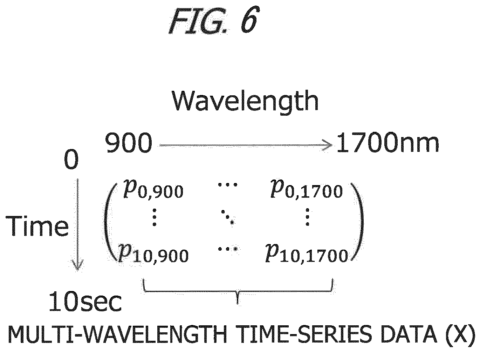

Here, an example of calculating the absorption spectrum according to this embodiment will be described. Fig. 6 illustrates an example of multi-wavelength time-series data X obtained by the spectrometer 240. As illustrated in Fig. 6, the multi-wavelength time-series data X are displayed in a matrix showing an amount of the light at each wavelength to be measured by the spectrometer 240. The amount of the light at each of the wavelengths is obtained at the measurement interval of the spectrometer 240 and arranged in time series. In the example of Fig. 6, the wavelengths of the light to be measured are divided into columns, and in each of the columns, the amount of light measured by the spectrometer 240 at the corresponding wavelength is arranged in time series. In the example above, the spectrometer 240 measures the light at each of the 62 different wavelengths within the wavelength range of 900 to 1700 nm for the period of 10 seconds, i.e., 200 times at the interval of 50 milliseconds, so as to obtain the optical spectrum at each of the 62 different wavelengths. Accordingly, the spectrometer 240 obtains data in the matrix arranged in 62 columns and 200 rows as the multi-wavelength time-series data indicating the pulsating signals at the plurality of wavelengths.

When the principal component analysis is performed on the multi-wavelength time-series data obtained by the spectrometer 240, the multi-wavelength time-series data X is expressed by a mathematical formula (2) below.

[Mathematical formula 2]

$$X = t_1 p_1^t + t_2 p_2^t + t_3 p_3^t + \cdots \quad \cdots \quad (2)$$

Here, in the principal component analysis, $t_i$ (i is a natural number) represents a score in a typically called ith principal component, and $p_i^t$ represents a loading in the typically called ith principal component. Each of the score and the loading is well-known, and thus a detailed description thereof will be omitted.

The measuring object of the absorption spectrum is the pulsation at a constant period, and thus the first principal component as a result of the principal component analysis may be highly attributed to the pulsation. Accordingly, in this embodiment, the first principal component is obtained first, and based on the obtained first principal component, the pulsating signal is calculated. Hence, in the multi-wavelength time-series data X expressed by the mathematical formula (2), the second and subsequent principal components are removed as noise. As a result, multi-wavelength time-series data X' is obtained, as expressed by a mathematical formula (3) below.
[Mathematical formula 3]

$$X' = t_1 p_1^t \quad \cdot \cdot \cdot \; (3)$$

With respect to the multi-wavelength time-series data X' that is removed noise, the variation width of the amount of light at each of the wavelengths is calculated as the standard deviation. Then, the variation width of the amount of light at each of the wavelengths is converted to the variation width of the absorbance by using the mathematical formula (1) and the time-averaged amount of light at each of the wavelengths, so that the absorption spectrum is calculated.

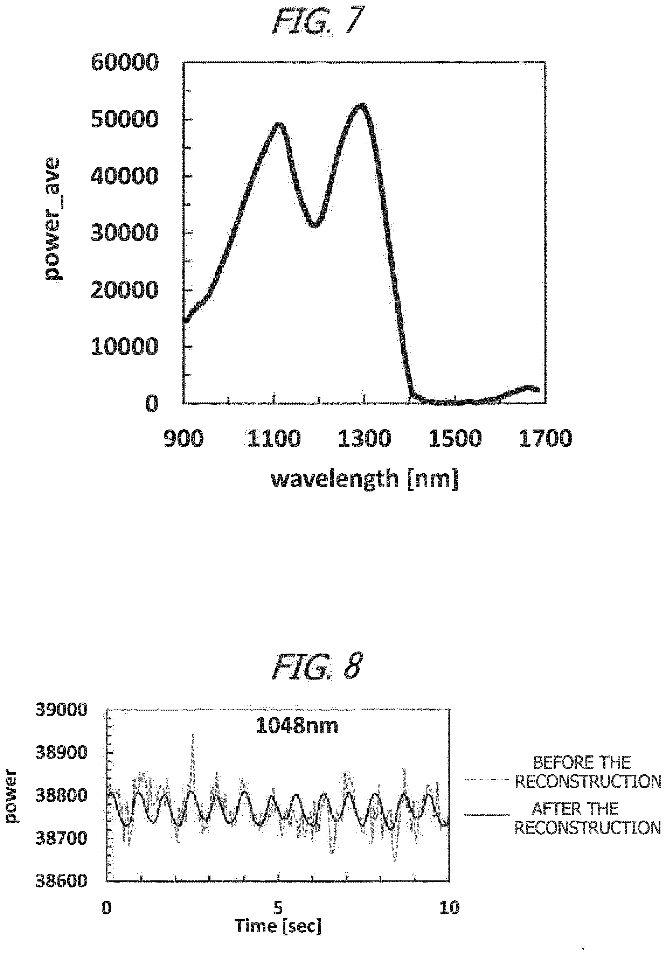

Next, an example of the absorption spectrum obtained by the calculation above will be described. Fig. 7 is a graph that illustrates a time-averaged transmitted light spectrum obtained over the measurement period, the transmitted light being received by the light receiver 230. In the graph of Fig. 7, each of the wavelengths is indicated on a horizontal axis, and the time-averaged value for amount of the transmitted light is indicated on a vertical axis. The amount of the transmitted light at each of the wavelengths periodically varies in accordance with the pulsation of the tube 270. Figs. 8 to 10 each illustrate a temporal variation in amount of the transmitted light (a pulsating signal) at one of these three wavelengths as an example: 1048 nm, 1138 nm, and 1195 nm. Figs. 8 to 10 each illustrate a temporal variation in amount of the transmitted light before the reconstruction (with a dotted line), and a temporal variation in amount of the transmitted light after the reconstruction (with a solid line). In the graph of each of Figs. 8 to 10, the elapsed time (in second) is indicated on a horizontal axis, and the amount of the transmitted light is indicated on a vertical axis. As illustrated in each of Figs. 8 to 10, the temporal variation in amount of the transmitted light before the reconstruction shows that the amount of the light irregularly varies. This is because the data related to the amount of the light includes noise. Conversely, with respect to the data on which the principal component analysis is performed for the reconstruction, the noise is removed from the data related to the amount of the light, and thus, the variation attributed to the pulsation is more clearly seen. The removal of noise by reconstructing the pulsating signal based on the light received by the light receiver 230 is performed at each of the three wavelengths, and is more significantly effective toward the lower wavelengths.

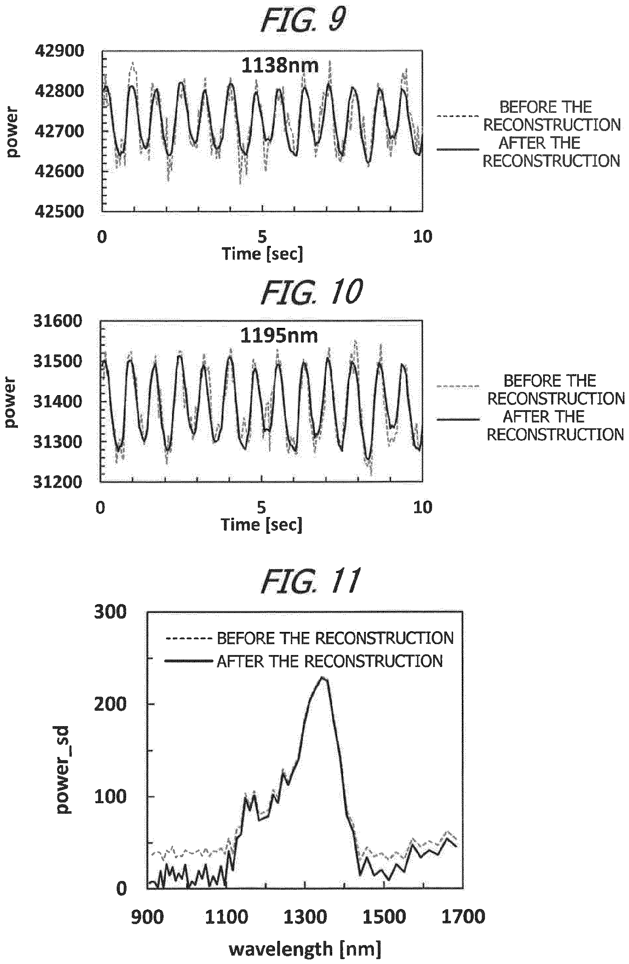

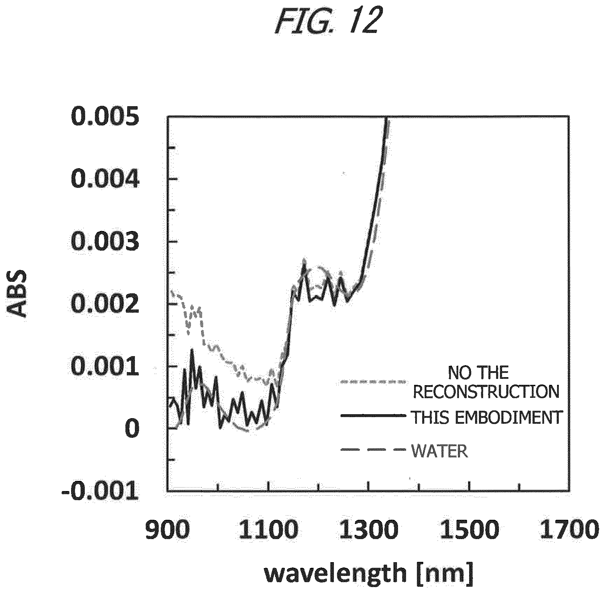

Fig. 11 illustrates the standard deviation of the temporal variation in amount of the light received by the light receiver 230 at each of the wavelengths, the standard deviation calculated as the variation width of the pulsating signal at each of the wavelengths. In Fig. 11, each of the wavelengths is indicated on a horizontal axis, and the standard deviation is indicated on a vertical axis. Additionally, Fig. 12 illustrates the absorption spectrum of the solution sent through the tube 270. The absorption spectrum is calculated using the time-averaged value for the amount of the transmitted light in Fig. 7, the standard deviation in Fig. 11, and the mathematical formula (1). In Fig. 12, each of the wavelengths is indicated on a horizontal axis, and the absorbance is indicated on a vertical axis. Fig. 12 also illustrates the absorption spectrum of the water to be sent through the tube, the water being separately measured.

The absorbance indicated by the absorption spectrum of the solution sent through the tube, which is calculated based on the variation in amount of the light in the pulsating signal before the reconstruction, is larger in value than the absorbance of the water within a wavelength range below 1100 nm. Conversely, the absorption spectrum of the solution sent through the tube, which is calculated based on the reconstructed pulsating signal according to this embodiment, significantly matches the absorption spectrum of the water.

This is because the standard deviation before the reconstruction includes not only the variation attributed to the pulsation but also the variation attributed to noise, causing the system to overestimate the variation width and obtain the absorbance calculated larger than the actual. On the other hand, the standard deviation after the reconstruction is reduced due to the removal of noise, causing the system to accurately estimate the variation attributed to the pulsation. Thus, the results according to the first example confirm that the system can noninvasively measure the pulsating signal of the solution sent through the tube and remove noise from the pulsating signal to accurately calculate the absorption spectrum of the solution.

Second example

Next, a second example according to this embodiment will be described. In the second example, the simulated blood vessel test system 2 described above is used. Note that, in the second example, a human finger is used instead of the tube 270, and light is applied to the human finger from the bundle fiber 220. The light transmitted through the human finger is received by the light receiver 230, and then, the spectrometer 240 measures a pulsating signal. Further, in the simulated blood vessel test system 2 of the second example, light is similarly applied to a non-pulsating simulated sample. The light transmitted through the sample is received by the light receiver 230, and then, the spectrometer 240 measures a pulsating signal. Then, as in the first example, an absorption spectrum is calculated from each of the pulsating signals, and these absorption spectra are compared with each other. As a result, a test is performed to see if the absorption spectrum of blood through the human finger, which is calculated according to this embodiment, is accurately obtained.

The light source 210 applies white light at a wavelength range of 900 to 1700 nm to the human finger, and the light transmitted through the human finger is received by the light receiver 230. The light received by the light receiver 230 is sent to the spectrometer 240. The spectrometer 240 measures the light received by the light receiver 230 at each of 62 different wavelengths within the wavelength range of 900 to 1700 nm for a period of 10 seconds, i.e., 200 times at an interval of 50 milliseconds, so as to obtain an optical spectrum at each of the 62 different wavelengths. Accordingly, as in the first example, the spectrometer 240 obtains multi-wavelength time-series data indicating the pulsating signals at the plurality of wavelengths.

As in the first example, a principal component analysis is similarly performed on the obtained multi-wavelength time-series data to obtain a reconstructed pulsating signal. With respect to the reconstructed pulsating signal, a variation width of the amount of light at each of the wavelengths, which is calculated as a standard deviation, is converted to a variation width of absorbance by using the mathematical formula (1) and a time-averaged amount of light at each of the wavelengths. As a result, an absorption spectrum of blood is calculated. Also, on a presumption of a non-pulsating human finger, only the filter paper 280 as light scattering medium is similarly measured instead of the human finger, and thus an absorption spectrum of the non-pulsating simulated sample is similarly calculated.

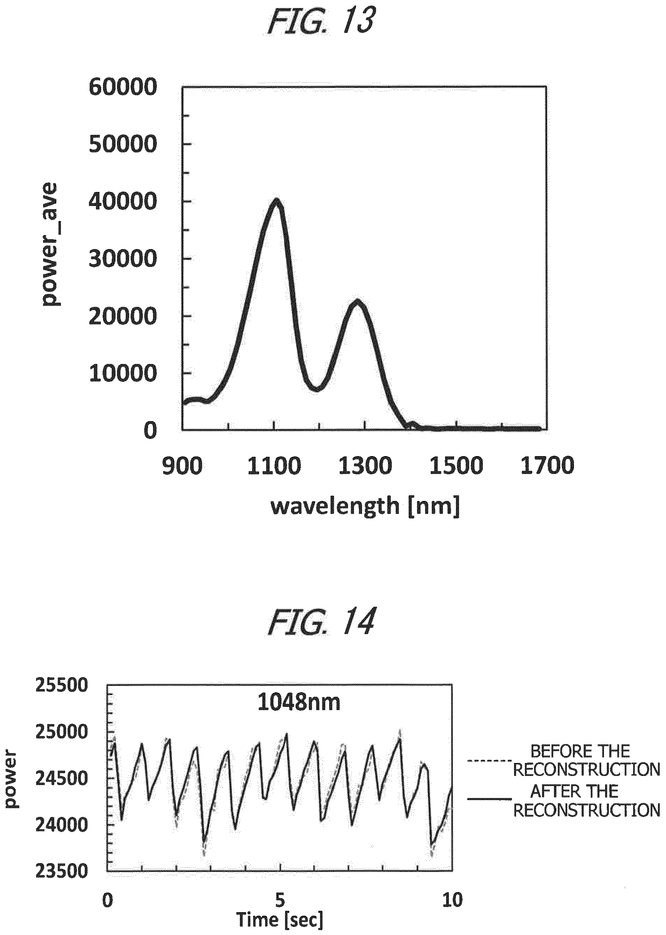

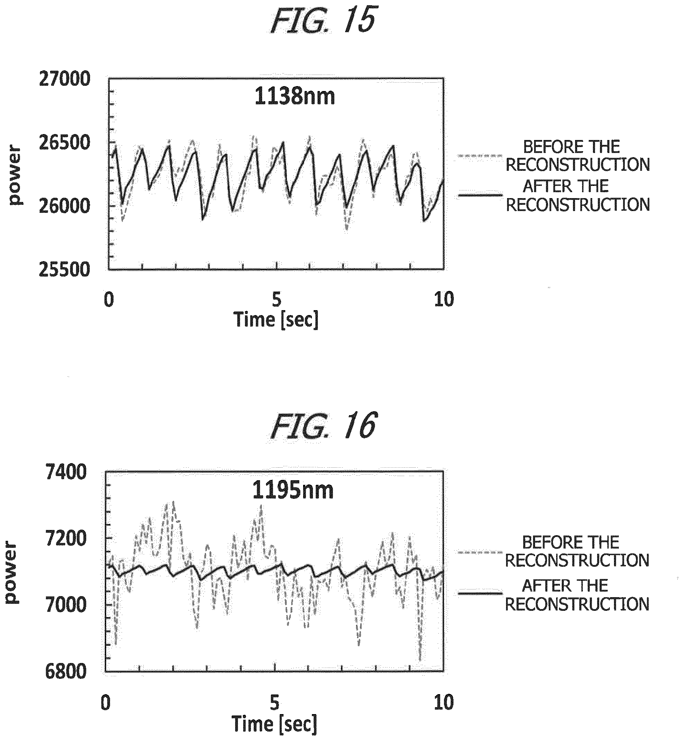

Fig. 13 is a graph that illustrates a time-averaged transmitted light spectrum, the light transmitted through the human finger and received by the light receiver 230. With respect to the transmitted light spectrum of the human finger, the amount of transmitted light at each of the wavelengths periodically varies in accordance with the pulsation. Figs. 14 to 16 each illustrate a temporal variation in amount of the transmitted light (a pulsating signal) at one of these three wavelengths as an example: 1048 nm, 1138 nm, and 1195 nm. Figs. 14 to 16 each illustrate a temporal variation in amount of the transmitted light before (or without) the reconstruction (with a dotted line), and a temporal variation in amount of the transmitted light after the reconstruction (with a solid line). In the graph of each of Figs.14 to 16, the elapsed time (in second) is indicated on a horizontal axis, and the amount of transmitted light is indicated on a vertical axis.

As illustrated in Figs. 14 to 16, noise is included in the temporal variation in amount of the transmitted light before the reconstruction, and conversely, with respect to the data on which the principal component analysis is performed for the reconstruction, the noise is removed so that a variation attributed only to the pulsation is apparently extracted. Also, as seen in a comparison between Figs. 14 to 16, the removal of noise is significantly effective at the wavelength of 1195 nm.

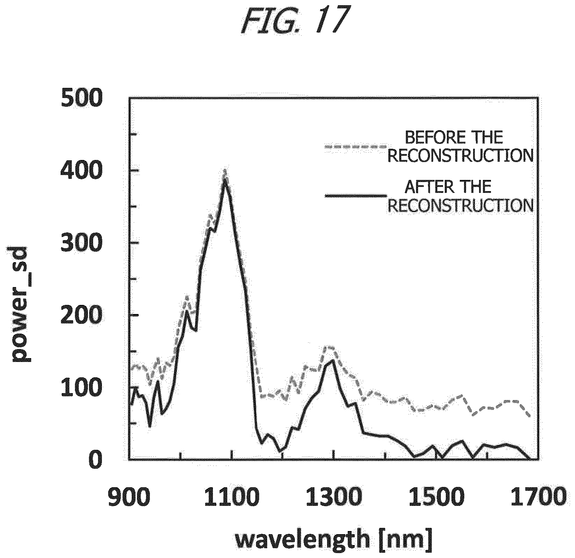

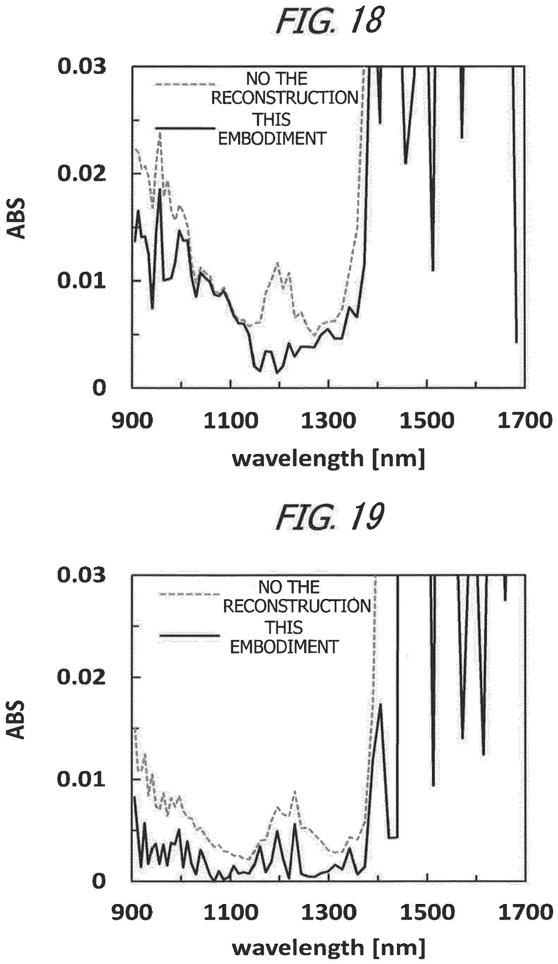

Fig. 17 illustrates the standard deviation of the temporal variation as variation width of pulsating signal in amount of the light received by the light receiver 230 at each of the wavelengths, the standard deviation being calculated before and after the reconstruction. In Fig. 17, each of the wavelengths is indicated on a horizontal axis, and the standard deviation is indicated on a vertical axis. Fig. 18 illustrates a result of the absorption spectrum of the human blood to be measured, the absorption spectrum calculated using the time-averaged value for the amount of the light in Fig. 13, the standard deviation in Fig. 17, and the above-mentioned mathematical formula (1). Additionally, as with the absorption spectrum of the human blood, Fig. 19 illustrates the absorption spectrum that is calculated in a case of applying the light to the paper filter 280 only. In the graph of each of Figs. 18 and 19, each of the wavelengths is indicated on a horizontal axis, and the absorbance is indicated on a vertical axis. Figs. 18 and 19 each illustrate an absorption spectrum based on the temporal variation in amount of the light before (or without) the reconstruction (with a dotted line), and an absorption spectrum based on the temporal variation in amount of the light after the reconstruction (with a solid line) .

As seen in Figs. 18 and 19, the absorption spectrum of blood representing the variation in amount of the light before the reconstruction is larger in overall value than the absorption spectrum of blood after the reconstruction, particularly exhibiting a peak at a wavelength in a vicinity of 1200 nm. Conversely, the absorption spectrum after the reconstruction does not exhibit the peak at the wavelength in the vicinity of 1200 nm. Further, in the case of the absorption spectrum measured only on the paper filter 280 that does not pulsate, the paper filter 280 is a non-pulsating object, so that the logical result is a zero spectrum, in other words, the absorbance is logically zero throughout the wavelength range. However, as illustrated in Fig. 19, the absorbance is not zero, and thus, the absorbance in Fig. 19 is clearly attributed to noise occurring in a process for the measurement of the absorption spectrum.

As has been described above, when the reconstruction is not performed on the data for the measured absorption spectra, the absorbance in each of the absorption spectra is overestimated. On the other hand, each of the absorption spectra calculated after the reconstruction is removed noise. Additionally, the peak at the wavelength in the vicinity of 1200 nm, which is exhibited in the data not reconstructed, is presumably a superficial peak generated by noise, and thus, the absorption spectrum before the reconstruction may not adequately reflect the absorption spectrum of blood from the living body. As with the first example, the second example confirms that reconstructing a pulsating signal according to this embodiment causes an absorption spectrum of blood to be further accurately calculated.

In view of the foregoing examples, the in-blood component calculation device 1 according to this embodiment for calculating an absorption spectrum of blood is configured to remove noise from a pulsating signal obtained by irradiating a living body with light at the plurality of wavelengths, so as to calculate an absorption spectrum of blood more accurately, as compared with the conventional technique.

This embodiment has been described above; however, a configuration of an in-blood component calculation device 1 or a method to calculate an absorption spectrum is not limited to those described in the foregoing embodiment, and may be modified in various manners within a range not deviating from a technical concept of the present invention.

For example, in this embodiment, light applied to a living body is monochromatic light transmitted through the living body, having at least two or more wavelengths selected from the wavelength range of 400 to 2500 nm. Alternatively, white light, including light at the whole or partial wavelength range from 400 to 2500 nm, may be applied, and the received light may be divided in accordance with the plurality of wavelengths to be measured. The white light applied to the living body has more preferably the wavelength range of 900 to 1700 nm, and still more preferably a wavelength range of 900 to 1300 nm. At these selected wavelength ranges, a pulsating signal that further accurately reflects pulsation of blood from the living body is measured. Then, it is possible to further effectively remove noise from the pulsating signal in a principal component analysis performed for reconstruction of the pulsating signal. The spectrometer 240 may perform spectrometry on the received light, preferably at an interval of 10 to 50 nm.

The multi-wavelength time-series data obtained based on light measured in this embodiment may represent any one of the following: an intensity of transmitted light through a measured part, an intensity of reflected light from the measured part, an intensity of scattered light from the measured part, or absorbance in the measured part. In other words, in the foregoing description, the light receiver 230 is presumed to receive transmitted light through the living body, but the light that the light receiver 230 receives is not limited to transmitted light. With a change in number or position of the light receiver 230, the light receiver 230 may receive transmitted light through the living body, reflected light from the living body, scattered light from the living body, or these plurality types of light. Then, the processing method described in the foregoing embodiment for calculating the absorption spectrum may be applied to any of these light received by the light receiver 230. With respect to the pulsating signal used for calculating the absorption spectrum, a pulsating signal based on the absorbance is preferably used, and more preferably a pulsating signal based on each of the intensity of transmitted light and the absorbance is used. At the principal component analysis on the multi-wavelength time-series data, as a preprocessing operation method for light signal at each wavelength, data centralization based on a time-averaged value is preferably performed. In this configuration, a center of variation in the intensity of light or the absorbance is obtained.

In this embodiment, in the principal component analysis, the multi-wavelength time-series data on the received light is reconstructed as a first principal component before a variation width in intensity of the light is calculated. As a calculating method for the variation width in intensity of the light, any one of the following on the time-series data may be used: a standard deviation, a difference between the maximum value and the minimum value, or an average of an absolute value of difference from the time-averaged value. As described in the foregoing examples, the standard deviation may particularly preferably be used for the calculation.

Also, at the calculation of absorption spectrum of blood in this embodiment, in order to remove noise unwanted for the calculation from the pulsating signal based on the received light, a processing method of an existing system may be concurrently used. For example, before or after the principal component analysis based on the present invention is performed, any one of the following processing methods may be used: applying frequency filtering to pick out only a specified frequency component; fitting a polynomial to the time-series data as a difference method so as to remove slow (low-frequency) drift; smoothing the data by applying a moving average filter or a Savitzky-Golay filter; or making a combination of these methods.

With respect to a spectrometer used to measure an absorption spectrum, the spectrometer may be the multi-channel Fourier-transform spectrometer used in this embodiment, or alternatively may be, for example, a single-channel Fourier-transform spectrometer, a multi-channel dispersive spectrometer, or a single-channel dispersive spectrometer.

[Reference Signs List]

1 in-blood component calculation device
10 controller
11 measuring unit
12 calculating unit
20 memory

30    irradiator
40    light receiver
50    display

**Claims**

1. An information processing method comprising:

   irradiating a living body with near-infrared light having a plurality of wavelengths;
   measuring a pulsating signal corresponding to each of the plurality of wavelengths based on the near-infrared light received from the living body;
   performing a principal component analysis on multi-wavelength time-series data including the measured pulsating signal corresponding to each of the wavelengths to reconstruct the pulsating signal; and
   calculating an absorption spectrum based on a variation width of the reconstructed pulsating signal.

2. The information processing method according to claim 1, wherein
   the principal component analysis is performed on the multi-wavelength time-series data to obtain a first principal component, and
   the pulsating signal corresponding to each of the wavelengths is calculated based on the obtained first principal component.

3. The information processing method according to claim 1 or 2, wherein
   the plurality of wavelengths are within a wavelength range from 400 nm to 2500 nm, both inclusive.

4. An information processing device comprising:

   a measuring unit that measures a pulsating signal corresponding to each of a plurality of wavelengths based on a near-infrared light having the plurality of wavelengths, the near-infrared light received from a living body by irradiating the living body with the near-infrared light; and
   a calculating unit that performs a principal component analysis on multi-wavelength time-series data including the measured pulsating signal corresponding to each of the wavelengths to reconstruct the pulsating signal, and calculates an absorption spectrum based on a variation width of the reconstructed pulsating signal.

5. The information processing device according to claim 4, wherein
   the calculating unit performs the principal component analysis on the multi-wavelength time-series data to obtain a first principal component, and calculates the pulsating signal corresponding to each of the wavelengths based on the obtained first principal component.

6. The information processing device according to claim 4 or 5, wherein
   the plurality of wavelengths are within a wavelength range from 400 nm to 2500 nm, both inclusive.

7. A program for causing a computer to perform:

   measuring a pulsating signal corresponding to each of a plurality of wavelengths based on a near-infrared light having the plurality of wavelengths, the near-infrared light received from a living body by irradiating the living body with the near-infrared light;
   performing a principal component analysis on multi-wavelength time-series data including the measured pulsating signal corresponding to each of the wavelengths to reconstruct the pulsating signal; and
   calculating an absorption spectrum based on a variation width of the reconstructed pulsating signal.

8. The program according to claim 7, wherein
   the program causes the computer to perform the principal component analysis on the multi-wavelength time-series data to obtain a first principal component, and calculate the pulsating signal corresponding to each of the wavelengths based on the obtained first principal component.

9. The program according to claim 7 or 8, wherein
   the plurality of wavelengths are within a wavelength range from 400 nm to 2500 nm, both inclusive.

# FIG. 1

*FIG. 2*

FIG. 3

# FIG. 4

```
        ┌─────────────┐
        │    START    │
        └──────┬──────┘
               │                               ┌─OP101
    ┌──────────▼──────────────────────────────┐
    │  IRRADIATE NEAR-INFRARED LIGHT AND       │
    │  RECEIVE TRANSMITTED LIGHT               │
    └──────────┬───────────────────────────────┘
               │                        ┌─OP102
        ┌──────▼──────────┐
        │   MEASURE THE    │
        │  PULSATING SIGNAL│
        └──────┬──────────┘
               │                        ┌─OP103
        ┌──────▼──────────┐
        │ REMOVE NOISE FROM│
        │ THE PULSATING SIGNAL│
        └──────┬──────────┘
               │                        ┌─OP104
        ┌──────▼──────────┐
        │  CALCULATE THE   │
        │ ABSORPTION SPECTRUM│
        └──────┬──────────┘
               │                        ┌─OP105
        ┌──────▼──────────┐
        │   DISPLAY THE    │
        │ ABSORPTION SPECTRUM│
        └──────┬──────────┘
               │
        ┌──────▼──────┐
        │     END     │
        └─────────────┘
```

# FIG. 5

FIG. 6

Wavelength

$$
\begin{array}{c}
900 \xrightarrow{\hspace{3cm}} 1700\text{nm}
\end{array}
$$

0

Time $\downarrow$

$$
\begin{pmatrix}
p_{0,900} & \cdots & p_{0,1700} \\
\vdots & \ddots & \vdots \\
p_{10,900} & \cdots & p_{10,1700}
\end{pmatrix}
$$

10sec

MULTI-WAVELENGTH TIME-SERIES DATA (X)

## FIG. 7

## FIG. 8

## FIG. 9

1138nm

BEFORE THE
RECONSTRUCTION
AFTER THE
RECONSTRUCTION

## FIG. 10

1195nm

BEFORE THE
RECONSTRUCTION
AFTER THE
RECONSTRUCTION

## FIG. 11

------- BEFORE THE RECONSTRUCTION
——— AFTER THE RECONSTRUCTION

## FIG. 12

Legend:
- NO THE RECONSTRUCTION
- THIS EMBODIMENT
- WATER

X-axis: wavelength [nm]
Y-axis: ABS

## FIG. 13

## FIG. 14

## FIG. 15

## FIG. 16

FIG. 17

## FIG. 18

## FIG. 19

## INTERNATIONAL SEARCH REPORT

| International application No. |
|---|
| PCT/JP2018/015866 |

A.  CLASSIFICATION OF SUBJECT MATTER
Int.Cl.  A61B5/1455(2006.01)i, G01N21/359(2014.01)i

According to International Patent Classification (IPC) or to both national classification and IPC

B.  FIELDS SEARCHED

Minimum documentation searched (classification system followed by classification symbols)
Int.Cl.  A61B5/1455, G01N21/359

Documentation searched other than minimum documentation to the extent that such documents are included in the fields searched

| | |
|---|---|
| Published examined utility model applications of Japan | 1922–1996 |
| Published unexamined utility model applications of Japan | 1971–2018 |
| Registered utility model specifications of Japan | 1996–2018 |
| Published registered utility model applications of Japan | 1994–2018 |

Electronic data base consulted during the international search (name of data base and, where practicable, search terms used)

C.  DOCUMENTS CONSIDERED TO BE RELEVANT

| Category* | Citation of document, with indication, where appropriate, of the relevant passages | Relevant to claim No. |
|---|---|---|
| Y | JP 2005-507729 A (NELLCOR PURITAN BENNETT INCORPORATED) 24 March 2005, paragraphs [0010]–[0036], fig. 1-8 & US 2003/0088164 A1, paragraphs [0010]–[0049], fig. 1-8 & WO 03/039340 A2 | 1-9 |

☒  Further documents are listed in the continuation of Box C.　　☐  See patent family annex.

| | |
|---|---|
| *　　Special categories of cited documents: | "T"　later document published after the international filing date or priority date and not in conflict with the application but cited to understand the principle or theory underlying the invention |
| "A"　document defining the general state of the art which is not considered to be of particular relevance | |
| "E"　earlier application or patent but published on or after the international filing date | "X"　document of particular relevance; the claimed invention cannot be considered novel or cannot be considered to involve an inventive step when the document is taken alone |
| "L"　document which may throw doubts on priority claim(s) or which is cited to establish the publication date of another citation or other special reason (as specified) | "Y"　document of particular relevance; the claimed invention cannot be considered to involve an inventive step when the document is combined with one or more other such documents, such combination being obvious to a person skilled in the art |
| "O"　document referring to an oral disclosure, use, exhibition or other means | |
| "P"　document published prior to the international filing date but later than the priority date claimed | "&"　document member of the same patent family |

| Date of the actual completion of the international search | Date of mailing of the international search report |
|---|---|
| 14 June 2018 (14.06.2018) | 10 July 2018 (10.07.2018) |

| Name and mailing address of the ISA/ | Authorized officer |
|---|---|
| Japan Patent Office<br>3-4-3, Kasumigaseki, Chiyoda-ku,<br>Tokyo 100-8915, Japan | |
| | Telephone No. |

Form PCT/ISA/210 (second sheet) (January 2015)

**INTERNATIONAL SEARCH REPORT**

International application No.

PCT/JP2018/015866

C (Continuation).    DOCUMENTS CONSIDERED TO BE RELEVANT

| Category* | Citation of document, with indication, where appropriate, of the relevant passages | Relevant to claim No. |
|---|---|---|
| Y | JP 7-508426 A (SCIENTIFIC GENERICS LIMITED) 21 September 1995, page 3, lower left column, lines 4-18, page 6, upper left column, line 9 to page 6, lower left column, the last line, fig. 1 & WO 93/07801 A1, specification, page 2, lines 1-20, page 12, line 19 to page 15, line 6, fig. 1 | 1-9 |
| Y | JP 2003-508744 A (CME TELEMETRIX INC.) 04 March 2003, claims 1-2, 4 & WO 01/16577 A1, claims 1-2, 4 | 1-9 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 3345481 B

- JP 4474145 B